Europäisches Patentamt

European Patent Office

Office européen des brevets

(11) Veröffentlichungsnummer: **0 297 619**
**A2**

(12) **EUROPÄISCHE PATENTANMELDUNG**

(21) Anmeldenummer: 88110611.6

(22) Anmeldetag: 02.07.88

(51) Int. Cl.4: **C12N 15/00 , C12P 13/22**

(30) Priorität: 02.07.87 DE 3721838

(43) Veröffentlichungstag der Anmeldung:
04.01.89 Patentblatt 89/01

(84) Benannte Vertragsstaaten:
AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Then, Johann, Dr.
Sulzbacher Weg 2
D-6238 Hofheim am Taunus(DE)
Erfinder: Hammann, Peter, Dr.
Mörikestrasse 6
D-6233 Kelkheim (Taunus)(DE)
Erfinder: Wöhner, Gerhard, Dr.
Flörsheimer Strasse 27
D-6093 Flörsheim am Main(DE)
Erfinder: Marquardt, Rüdiger, Dr.
Günthersburgallee 69
D-6000 Frankfurtam Main(DE)
Erfinder: Mixich, Johann, Dr.
An der Ziegelei 14
D-6233 Kelkheim (Taunus)(DE)

(54) Verfahren zur Herstellung von L-Phenylalanin aus Benzylidenhydantoin.

(57) L-Phenylalanin kann nach der Hydrolyse von Benzylidenhydantoin als Vorstufe durch anschließende Inkubation der anfallenden ungereinigten Reaktionslösung unter Zugabe eines Aminogruppendonators mit einem gentechnisch manipulierten E. coli ATCC 11303 hergestellt werden.

EP 0 297 619 A2

# Verfahren zur Herstellung von L-Phenylalanin aus Benzylidenhydantoin

Die Herstellung von L-Aminosäuren durch Biotransformation mit Transaminasen ist an sich bekannt. Nach der Europäischen Patentanmeldung erfolgt die Herstellung von L-Aminosäuren derart, daß α-Ketosäuren mit L-Asparaginsäure in Gegenwart einer Transaminase umgesetzt werden, die aus E. coli isoliert wurde. Die Selektion und Mutation von Mikroorganismen aus der Reihe E.coli, Paracoccus denitrificans, Torula, Rhodotorula und Streptomyces zur Herstellung von L-Phenylalanin aus Phenylbrenztraubensäure wird in der Deutschen Patentanmeldung DE 3 423 936 dargestellt.

Phenylpyruvat ist jedoch ein relative teures Ausgangsprodukt. Um daher die Biotransformation von Phenylpyruvat zu L-Phenylalanin wirtschaftlicher zu gestalten, ist es wünschenswert von einer preiswerten Vorstufe des Phenylpyruvat ausgehen zu können. Eine derartige Vorstufe ist das Benzylidenhydantoin, aus dem Phenylbrenztraubensäure mit Hilfe einer einfachen alkalischen Hydrolyse hergestellt werden kann.

Der Einsatz der ungereinigten, neutralisierten Reaktionslösung nach der Benzylidenhydantoin-Hydrolyse war bislang aufgrund der Nebenprodukte und der hohen Salzkonzentration für fermentative Zwecke zur Herstellung von Phenylalanin nur mit - schlechter Ausbeute (DOS 34 27 495) möglich. Nicht-halophile Mikroorganismen, zu denen auch E. coli zählt, werden in ihrem Wachstum und ihren enzymatischen Reaktionen schon bei Salzkonzentrationen von 2 % wesentlich gehemmt [Larvon, H., Advan. Microbiol. Physiol. 1, 97 (1967)].

Nach der Benzylidenhydantoin-Hydrolyse liegen Reaktionslösungen mit Salzkonzentrationen von 9 bis 11 % vor. Die anschließende Aufreinigung des Phenylpyruvats ist jedoch sehr kostenntensiv.

Überraschenderweise kann mit Hilfe eines mit einem für aromatische Transaminase kodierenden Plasmid transformierten Mikroorganismus, der sich als halophil erwies und gemäß der Deutschen Patentanmeldung P 36 31 829.9 oder P 37 13 755.2 hergestellt werden kann, nach der alkalischen Hydrolyse des Benzylidenhydantoin die Biotransformation des Hydrolyseprodukts Phenylpyruvat zu Phenylalanin ohne Zwischenreinigung ausgeführt werden.

Die Erfindung betrifft somit ein Verfahren zur Herstellung von L-Phenylalanin durch Biotransformation von Phenylpyruvat mit E. coli ATCC 11303, transformiert mit einem extrachromosomalen Element, welches das aus E. coli ATCC 11303 isoliert tyrB-Gen enthält, das dadurch gekennzeichnet ist, daß

a) Benzylidenhydantoin alkalisch hydrolysiert wird und

b) die ungereinigte Reaktionslösung mit dem genannten transformierten E. coli in Gegenwart eines Aminogruppendonators inkubiert wird.

Im folgenden wird die Erfindung detailliert, insbesondere in ihren bevorzugten Ausführungsformen, beschrieben. Ferner wird die Erfindung in den Patentansprüchen definiert.

Benzylidenhydantoin wird nach an sich bekannten Methoden alkalisch gespalten. In Bezug auf die eingesetzte Menge an Benzylidenhydantoin wird bevorzugt mit Natronlauge in 3 bis 7 fachem Überschuß unter Rückfluß über einen Zeitraum von 30 bis 80 Minuten hydrolysiert. Nach Beendigung der Reaktion wird die alkalische Reaktionslösung neutralisiert, bevorzugt mit konzentrierter Salzsäure. Man erhält so eine Lösung, die außer 8% Phenylpyruvat etwa 9 bis 11 % NaCl bzw. die gleiche Menge eines entsprechenden anderen Salzes enthält. Weiterhin befinden sich in der Lösung bis zu 5 % Harnstoff, Phenylessigsäure, Benzaldehyd und weitere nicht näher identifizierte Dimerisierungsverbindungen der entsprechenden Zwischenprodukte. Das Phenylpyruvat in diesem Reaktionsgemisch kann ohne weitere Aufreinigung mit dem gemäß der Deutschen Patentanmeldung P 36 31 829.9 transformierten E. coli ATCC 11303 zu Phenylalanin umgesetzt werden. Alternativ kann E. coli ATCC 11303 mit einem Plasmid transformiert werden, das nicht nur das tyrB-Gen enthält, sondern auch das für Aspartase kodierende aspA-Gen. Ein derartiges Plasmid ist nach der Deutschen Patentanmeldung P 37 13 755.7 herzustellen und wird dann in E. coli ATCC 11303 eingeschleust.

Ein tyrB Gen enthaltendes Plasmid kann gemäß Deutscher Patentanmeldung P 36 31 829.9 wie folgt hergestellt werden: DNA wird aus E. coli ATCC 11303 isoliert. Nach partieller Verdauung der DNA von E. coli ATCC 11303 werden die entstandenen Fragmente, die sich in einem Größenbereich von ca. 20 bis 30 kb bewegen, in ein Cosmid mit einem Replikon, das einen weiten Wirtsbereich vermittelt, ligiert und in die Köpfe des λ-Phagen verpackt. Bevorzugt wird das Cosmid pIMS6026 verwendet. Das Cosmid pIMS 6026 leitet sich von dem Cosmid pLAFR1 (ATCC 37167) dadurch ab, daß in die einzige EcoRI Schnittstelle des Cosmids pLAFR1 das handelsübliche EcoRI Fragment (Pharmacia, Upsala, Schweden) kloniert wurde, auf dem das Kanamycin-Resistenzgen das Transposons Tn 903 liegt. Durch Verdauung mit BamHI und anschließender Religation kann der größte Teil des Kanamycin-Gens deletiert werden, so daß ein kurzes Stück DNA als Insertion zurückbleibt, in der eine Bam HI-Schnittstelle von 2 EcoRI-Schnittstellen flankiert wird. Diese Bam HI-Schnittstelle, die auf dem Cosmid pLAFR1 nicht vorhanden ist, kann für die Klonierung verwendet werden. Durch Inkubation der verpackten Cosmide mit einer entsprechend vorbereiteten E. coli DG 30 Suspension werden die Cosmide in den Mikroorganismus eingeschleust. E.coli DG 30 hat eine Defizienz in den drei Transaminasen von aspC,ilvE und tyrB. Der Stamm wächst daher auf Vollmedium zwar problemlos an, muß aber bei Wachstum auf Minimalmedium mit den entsprechenden Aminosäuren von außen versorgt werden, da er sie nicht selbst synthetisieren kann. Bei geeigneter Wahl des Mediums kann mit Hilfe des Stamms untersucht werden, ob eine von außen aufgenommene DNA den chromosomalen Defekt für eine bestimmte Transaminase komplementieren kann. Die Einschleusung des tyrB-Gens detektiert man durch Wachstum des E. coli DG 30 auf einem tyrosinfreien Minimalmedium. Auf diesem Medium können nur Klone anwachsen, die durch Aufnahme von aspC, tyrB und ilvE enthaltender DNA, die ursprünglich aus dem Stamm E. coli ATCC 11303 stammt, ihren chromosomalen Defekt für die Synthese von Tyrosin komplementierern können. Die drei Transaminasen, die von den Genen aspC, tyrB und ilvE codiert werden, unterscheiden sich in ihrer Substratspezifität, wenngleich sie alle drei in der Lage sind, Tyrosin zu bilden.

Die aromatische Transaminase, die von dem Gen tyrB codiert wird, kann beispielsweise kein Isoleucin aus der Ketovorstufe bilden, wohl aber Leucin in guten Ausbeuten aus der entsprechenden Ketovorstufe synthetisieren. Die Transaminase, die von dem Gen aspC codiert wird, kann weder Isoleucin bilden noch ist sie effizient in der Umsetzung zu Leucin.

Dementsprechend können die einzelnen Klone durch Wachstum auf einem Minimalmedium, das bis auf eine für das jeweilige Gen charakteristische Aminosäure mit für den Stoffwechsel essentiellen Aminosäuren supplementiert ist, hinsichtlich der enthaltenen Transaminase unterschieden werden. Auf diese Weise werden Klone des DG 30 Stammes, die das tyrB-Gen enthalten, heraus selektioniert.

Es muß nun überprüft werden, ob die Klone auch wirklich das tyrB Gen besitzen. Hierzu muß die Plasmid-DNA aus den Klonen isoliert werden. Die Isolierung aus dem Stamm DG 30 gelingt jedoch nur mit Schwierigkeiten. Es kann zwar Plasmid-DNA erhalten werden, jedoch nur in geringen Ausbeuten. Nach Minilyse wird daher ein E. coli Stamm mit den Cosmid-DNAs aus den interessanten Klonen transformiert, aus dem anschließend die eingeschleuste DNA in guten Ausbeuten reisoliert werden kann. E. coli DH1 (ATCC 33849) ist hierzu insbesondere geeignet.

In dem nächsten Schritt wird die reisolierte Cosmid DNA mit einem Vektor hoher Kopienzahl ligiert. Aus der chromosomalen Genkarte des Stammes E. coli K12 ist bekannt, daß das tyrB-Gen auf einem ClaI Fragment lokalisiert ist. Es bestand die Möglichkeit, daß dies auch bei E. coli ATCC 11303 der Fall ist. Aus diesem Grund wird als Vektor bevorzugt ein Multi-Copy-Plasmid mit einer ClaI-Schnittstelle eingesetzt, insbesondere bevorzugt das in seiner Sequenz bekannte pAT153 (Winnacker E.L., Gene und Klone, VCH-Verlagsgesellschaft, Weinheim).

Die Cosmid-DNA und der Vektor werden mit dem Restriktionsenzym ClaI vollständig verdaut. Die beiden DNAs werden zusammengegeben, miteinander ligiert und kompetente Zellen eines Wirtsorganismus, in dem die Phenylalaninproduktion erhöht werden soll, damit transformiert. Bevorzugt werden Enterobakterien eingesetzt, insbesondere E. coli, wobei E. coli ATCC 11303 sowie seine Mutanten und Varianten besonders bevorzugt sind. Resistente Kolonien werden über Ampicillin selektioniert und anhand von Marker-Inhibierung durch "replica plating" auf Tetracyclin-Platten auf Einbau getestet. Aus den entsprechenden Kolonien wird durch Minilyse Plasmid-DNA isoliert und durch vollständige Verdauung mit dem Restriktionsenzym ClaI das Vorhandensein von ClaI Fragmenten in dem Vektor überprüft.

Durch Restriktionsanalyse wird sichergestellt, daß alle in dem ursprünglichen DNA-Abschnitt enthaltenen ClaI Fragmente auf diese Art subkloniert worden sind. Klone, die jeweils eins dieser definierten ClaI Fragmente enthalten, werden schließlich mittels Aspartat-Phenylpyruvat Aminotransferase (APPAT)-Test auf die Aktivität der aromatischen Transaminase, also des Genprodukts von tyrB, getestet. Auf diese Weise läßt sich eine Zunahme der tyrB-Aktivität um den Faktor 5 bis 10 erreichen. Durch Agarose-Gelelektrophorese kann gezeigt werden, daß in dem Vektor des Wirtsstammes ein ca. 2,7 MD großes ClaI Fragment enthalten ist.

Ein Plasmid, das sowohl das tyrB-Gen als auch das aspA-Gen aus E. coli enthält, kann nach der Deutschen Patentanmeldung P 37 13 755.7 wie folgt hergestellt werden:

Zur Isolierung der aspA und tyrB-Gene aus Plasmiden werden bevorzugt die bereits beschriebenen bzw. vorgeschlagenen Multi-Copy-Plasmide pGS73 [Guest J.R. et al., J. Gen. Microbiol. 130, 1271-1278 (1984)] und pIMS6056 [Deutsche Patentanmeldung P 36 31 829.9] verwendet. Mit Hilfe von Restriktionsenzymen wird das Aspartase kodierende Gen aspA aus pGS73 isoliert. Das Plasmid wird vorzugsweise mit dem Restriktionsenzym BclI oder einer Kombination der Enzyme SphI und SalI geschnitten, so daß man das Aspartase-Gen auf einem 2,9 kb oder 6,2 kb Fragment erhält. Das tyrB-Gen ist nach Spaltung mit dem Restriktionsenzym Sall oder einer Kombination der Enzyme Sall und SphI aus dem Plasmid pIMS6056 erhältlich. Darüberhinaus sind die aspA- und tyrB-Gene der Plasmide pGS73 und pIMS6056 durch Spaltung mit einer Reihe weiterer Restriktionsendonukleasen zu isolieren [Guest, J.R. et al, J. Gen. Microbiol. 130, 1271-1278 (1984); Deutsche Patentanmeldung P 36 31 829.9].

Nach der Isolierung beider Gene werden diese auf ein geeignetes Replikon kloniert, beispielsweise das E. coli Multi-Copy-Plasmid pBR322 [Bolivar F. et al., Gene 2, 95-113 (1977)] oder das Plasmid RSF1010 [Guerry P. et al., J. Bacteriol. 117, 619-630 (1974)], das sich gegenüber pBR322 durch ein breiteres Wirtsspektrum auszeichnet, sowie deren Derivate. Prophagen-DNS kann ebenfalls als Replikon eingesetzt werden, z.B. das P1-Prophagengenom. Bevorzugt werden zur Konstruktion der erfindungsgemäßen Hybridplasmide in E. coli die Plasmide pGS73 und pIMS6056 verwendet. Die isolierten Gene können dann jeweils auf das Plasmid gebracht werden, auf dem das Gen noch nicht enthalten ist.

Die Ablesung beider Gene erfolgt von zwei verschiedenen Promotoren aus, die den entsprechenden chromosomalen aspA-und tyrB-Genen jeweils vorangeschaltet sind. Alternativ können beide Gene auch gemeinsam oder getrennt mit Hilfe eines starken fremden Promotors, der konstitutiv oder reguliert aktiv ist, transkribiert werden.

Es ist auch möglich, daß das aspA- bzw. tyrB-Gen jeweils in zwei verschiedene kompatible Plasmide kloniert wird, die in der Mikroorganismen-Zelle später nebeneinander bestehen können.

Die Hybridplasmide, die die genannten Gene zusammen oder einzeln kloniert enthalten, werden in E. coli ATCC 11303 transformiert. Ampicillinresistente Klone werden durch Restriktionsanalyse auf das Vorhandensein des gewünschten Hybridplasmides untersucht. Aspartase und aromatische Transaminase kodierende Klone können über viele Passagen ohne Verlust der jeweiligen Hybridplasmide erfindungsgemäß zur L-Phenylalaninproduktion aus Phenylpyruvat und Ammoniumfumarat bzw. Fumarsäure oder Fumarat und Ammoniumionen oder Harnstoff eingesetzt werden.

Die transformierten Stämme werden in einem entsprechend guten Wachstumsmedium bis zu einem Feuchtgewicht von etwa 4 bis 10 g/l Nährlösung angezogen. Die Zellen werden dann in der Nährlösung oder von der Nährlösung abgetrennt zur Aminierung der Ketosäuren eingesetzt. Die Transaminierung kann mit ganzen oder auch mit aufgeschlossenen Zellen durchgeführt werden, wobei die üblichen Aufschlußmethoden angewendet werden. Aus Gründen der Arbeitserleichterung wird bevorzugt mit intakten Zellen gearbeitet. Es ist weiterhin möglich, die Mikroorganismen in fixierter Form einzusetzen. Für die Fixierung kommen die bekannten Verfahren in Betracht, vorteilhaft die Methoden nach den Deutschen Offenlegungsschriften 32 37 341 und 32 43 591.

Je nach Menge der Mikroorganismen kann die dem Reaktionsansatz zugegebene enzymatische Aktivität in weiten Bereichen schwanken. Zweckmäßig liegt sie zwischen 100 und 30.000 μmol/min·l. Vorzugsweise enthält der Ansatz Zellmengen mit einer Enzymaktivität von 10.000 bis 20.000 μmol/min·l.

Als Aminogruppendonator finden Asparagin, Asparaginsäure, Glutamin und Glutaminsäure Anwendung oder Ammoniumfumarat oder Fumarat bzw. Fumarsäure mit Ammoniumionen oder Harnstoff. Diese Vorstufen werden in Form ihrer freien Säure oder geeigneten Salze (entsprechend dem verwendeten Medium) in äquimolaren Mengen bzw. im Überschuß zur α-Ketosäure eingesetzt. Verhältnisse von 1:1 bis 5:1, vorteilhaft 1:1 bis 2:1, haben sich bewährt.

Unter Zugabe eines für die Zellen physiologischen Puffers wird vorteilhaft bei einem pH-Wert zwischen 5 und 9, insbesondere zwischen 7 und 8,5, gearbeitet. Es ist außerdem zweckmäßig, die Transaminierung in einem Temperaturbereich von 20 bis 65°C durchzuführen. Bei niedrigeren Temperaturen verläuft die Enzym- Reaktion zunehmend langsamer, während das Enzym bei höheren Temperaturen fortschreitend desaktiviert wird. Die Reaktionszeit beträgt 1 bis 90 Stunden, vorzugsweise 2 bis 40 Stunden.

Es hat sich besonders bewährt, die Mikroorganismen vor bzw. während der Transaminierungsreaktion zu permeabilisieren. Dies kann durch Zugabe geeigneter Agenzien, wie Toluol, Cetyltrimethylammoniumbromid, Dimethylsulfoxid etc., zum Inkubationsmedium erfolgen.

Hohe Umsatzraten in kurzer Zeit werden insbesondere dann erreicht, wenn der beschriebene Reaktionsansatz während der Inkubation mit den transformierten E. coli-Stämmen begast wird, wie auch schon in der Deutschen Patentanmeldung P 36 13 952.1 dargestellt. Die Begasung erfolgt im Bereich von 0,2 bis 15 VVm, vorteilhaft im Bereich von 1 bis 5 VVm. Es können grundsätzlich alle Gase eingesetzt werden, die die Enzymaktivität des Mikroorganismus nicht wesentlich heruntersetzen. Geeignet sind beispielsweise Preßluft, reiner Sauerstoff, Stickstoff aber auch verschiedene Edelgase, wie Helium, Neon, Argon oder Krypton. Aufgrund des günstigen Preises und ihrer Zugänglichkeit sind Preßluft und reiner Stickstoff bevorzugt.

Die anschließenden Beispiele dienen dazu, die vorgestellte Erfindung weiter zu illustrieren. Prozentangaben beziehen sich, wenn nicht anders angegeben, auf das Gewicht.

## Beispiel 1

Transformierte E. coli Stämme, die gemäß den Beispielen der Deutschen Patentanmeldungen 36 31 829.9 oder 37 13 755.7 hergestellt worden waren, wurden bei 37° C in folgender Nährlösung submers kultiviert:

$Na_2HPO_4$     3,5g/l
$KH_2PO_4$     1,8 g/l
$(NH_4)_2HPO_4$     12 g/l
$(NH_4)_2SO_4$     6 g/l
$MgSO_4$     0,2 g/l
Hefeextrakt     1 g/l
Glucose     5 g/l
pH 7,0 wurde mit NaOH eingestellt.

Der genetisch manipulierte Stamm wurde in 100 ml des Wachstumsmediums 5 h bei 37° C geschüttelt. Anschließend wurde ein 10 l Fermenter angeimpft. Nach 4 h Wachstum wurde mit einer exponentiell ansteigenden Rate Glucose zugefüttert. Es wurde mit einer Zufütterungsrate von 0,3 g/l•h begonnen und nach 8 Stunden mit 8 g/l•h weitergefüttert. Nach insgesamt 12 Stunden Wachstum wurde eine Biomasse von 10 g/l Trockengewicht erreicht mit einer Enzymaktivität von 14000 U/l.

Die Transaminaseaktivität wurde durch einen APPAT-Test (Aspartat-Phenylpyruvat-Aminotransferase Assay, Sigma Test-Kit GO390) bestimmt.

## Beispiel 2

Aus Benzylidenhydantoin wurde durch alkalische Hydrolyse Phenylpyruvat hergestellt. Dann wurde 1,2 Mol Benzylidenhydantoin in einer Lösung von 6 Mol Natriumhydroxyd in 3 l Wasser auf 100° C erhitzt und 60 Minuten bei dieser Temperatur gerührt. Die Lösung wurde auf Raumtemperatur abgekühlt und mit 60 ml (30 Gew.-%) Salzsäure auf einen pH-Wert von 7,5 gebracht.

## Beispiel 3

3 l der Lösung aus Beispiel 2 wurden ohne weitere Reinigung mit 1,4 mol Asparaginsäure, 5 g $MgCl_2$ und 10 ml (R)Tween 80 versetzt. Dan wurden Zellen, die wie in Beispiel 1 hergestellt wurden, mit einer Transaminase-Aktivität von 25 000 μmol/min. hinzugefügt und mit 50 millimolarem Phosphatpuffer (pH 7,4) auf 7 l aufgefüllt. Nach einer Inkubationszeit von 15 Stunden bei 37° C und einer Begasung von 1,5 VVm Preßluft konnten in dem Reaktionsgemisch 26 g/l L-Phenylalanin gemessen werden. Die Lösung wurde filtriert und das L-Phenylalanin wurde nach konventionellen Methoden auskristallisiert und gereinigt.

## Beispiel 4

E. coli wurde wie in Beispiel 1 angezogen und Zellen mit einer Transaminaseaktivität von insgesamt 1500 μmol/min. ohne wietere Reinigung direkt in folgende Reaktionslösung gebracht:
300 mmol/l Asparaginsäure•Na-Salz,
1,2g/l$MgCl_2$•$6H_2O$,
1 ml/l Polyoxyethylen-Sorbitan-Monooleat ( Tween 80).

Es wurden zwei Ansätze verglichen. Ein Ansatz enthielt zusätzlich 220 mmol gereinigtes Phenylpyruvat•Na-Salz (Riedel-de Haen) und der andere zusätzlich eine wie in Beispiel 3 hergestellte neutralisierte Phenylpyruvatlösung in entsprechender Menge. Es wurde bei 50° C 7 Stunden mit einer Begasungsrate von 1 VVm Preßluft belüftet. Mit Hilfe der HPLC-Analytik wurde der Phenylalaningehalt gemessen. Der Ansatz mit gereinigtem Phenylpyruvat enthielt 215 mmol/l L-Phenylalanin und der andere Ansatz 210 mmol/l L-Phenylalanin.

## Ansprüche

1. Verfahren zur Herstellung von L-Phenylalanin durch Biotransformation von Phenylpyruvat mit E. coli ATCC 11303, transformiert mit einem extrachromosomalen Element, welches das aus E. coli ATCC 11303 isolierte tyrB-Gen enthält, dadurch gekennzeichnet, daß
a) Benzylidenhydantoin alkalisch hydrolysiert wird und
b) die ungereinigte Reaktionslösung mit dem genannten transformierten E. coli
in Gegenwart eines Aminogruppendonators inkubiert wird.

2. Verfahren nach Anspruch 1, dadurch gekennzeichnet, daß bei pH-Werten von 5 bis 9 inkubiert wird.

3. Verfahren nach Anspruch 2, dadurch gekennzeichnet, daß bei pH-Werten von 7 bis 8,5 inkubiert wird.

4. Verfahren nach einem oder mehreren der Ansprüche 1 bis 3, dadurch gekennzeichnet, daß bei einer Temperatur von 20 bis 65° C inkubiert wird.

5. Verfahren nach einem oder mehreren der Ansprüche 1 bis 4, dadurch gekennzeichnet, daß die Reaktionslösung während der Inkubation begast wird.

6. Verfahren nach einem oder mehreren der Ansprüche 1 bis 5, daudrch gekennzeichnet, daß E. coli ATCC 11303 zusätzlich zu dem tyrB-Gen noch ein aus E. coli isoliertes aspA-Gen enthält.